(19) **Europäisches Patentamt**

**European Patent Office**

**Office européen des brevets**

(11) **EP 1 660 929 B1**

(12) **FASCICULE DE BREVET EUROPEEN**

(45) Date de publication et mention
de la délivrance du brevet:
**29.11.2006 Bulletin 2006/48**

(51) Int Cl.:
**G02C 7/02** (2006.01)

(21) Numéro de dépôt: **04786292.5**

(22) Date de dépôt: **11.08.2004**

(86) Numéro de dépôt international:
**PCT/FR2004/002120**

(87) Numéro de publication internationale:
**WO 2005/022241 (10.03.2005 Gazette 2005/10)**

(54) **SYSTEME OPTIQUE DE COMPENSATION ACCOMMODATIVE**

**OPTISCHES AKKOMODATIVES KOMPENSATIONSSYSTEM**

**OPTICAL ACCOMMODATIVE COMPENSATION SYSTEM**

(84) Etats contractants désignés:
**AT BE BG CH CY CZ DE DK EE ES FI FR GB GR
HU IE IT LI LU MC NL PL PT RO SE SI SK TR**

(30) Priorité: **26.08.2003 FR 0310171**

(43) Date de publication de la demande:
**31.05.2006 Bulletin 2006/22**

(73) Titulaire: **ESSILOR INTERNATIONAL (Compagnie
Générale
d'Optique)
F-94220 Charenton-le-Pont (FR)**

(72) Inventeurs:
• **CHAUVEAU, Jean-Pierre
F-75013 Paris (FR)**
• **LE SAUX, Gilles
F-75010 Paris (FR)**
• **DECRETON, Bruno
F-94220 Charenton Le Pont (FR)**

(74) Mandataire: **Chauvin, Vincent et al
CORALIS
Conseils en Propriété Industrielle
85 boulevard Malesherbes
75008 Paris (FR)**

(56) Documents cités:
**US-A- 2 164 801          US-A- 3 027 803**

## Description

### DOMAINE TECHNIQUE AUQUEL SE RAPPORTE L'INVENTION

La présente invention se rapporte de manière générale à la compensation ou correction des troubles de la vision, et plus particulièrement aux cas dans lesquels la fonction accommodative de l'oeil est déficiente. Elle concerne concrètement un système optique mis en oeuvre pour compenser cette déficience, ainsi qu'un équipement visuel incorporant ce système.

Plus spécifiquement encore, mais non exclusivement, elle touche au domaine de l'implantation de lentilles intraoculaires artificielles et concerne alors un système optique de correction de l'oeil aphake.

### ARRIERE-PLAN TECHNOLOGIQUE

De manière générale, un système optique de compensation ou correction visuelle peut faire intervenir deux principaux types de lentilles.

Une lentille d'ouverture est conçue pour exercer la fonction optique qui lui confère son pouvoir correcteur ou compensateur dans ou contre l'oeil. Il s'agit typiquement d'une lentille intraoculaire ou d'une lentille de contact cornéenne. La fonction optique qu'exerce sur l'oeil une telle lentille d'ouverture dépend de l'ouverture de la pupille, mais est indépendante de la direction de regard.

Une lentille de champ est au contraire conçue pour exercer sa fonction optique à distance de l'oeil. Il s'agit typiquement d'un verre de lunettes monté sur une monture placée sur le nez du porteur. Il peut aussi s'agir d'un monocle. La fonction optique qu'exerce sur l'oeil une telle lentille de champ dépend de la direction de regard, mais est indépendante de l'ouverture de la pupille.

Ces deux types de lentilles sont utilisés le plus souvent de façon alternative, c'est-à-dire dans des systèmes optiques de correction incorporant des lentilles appartenant à l'un des deux types précités, à l'exclusion de lentilles de l'autre type. Plus rarement, dans des cas spécifiques qui seront évoqués ultérieurement, il peut arriver que les deux types de lentilles soient combinés au sein d'un même système optique de correction. La présente invention concerne précisément un tel système hybride et propose son utilisation dans le contexte suivant.

Parmi les différents troubles de la vision susceptibles d'affecter l'oeil, on s'intéresse plus particulièrement, dans le cadre de la présente invention, à la perte d'accommodation, partielle ou totale.

L'accommodation est le processus par lequel l'oeil met au point sur les objets plus ou moins rapprochés et permet ainsi de voir des objets successivement et nettement, alors qu'ils sont situés à des distances différentes, appelées cliniquement distances de visée ou de travail. On distingue ainsi classiquement trois zones de visée : la zone de vision de loin correspondant à des distances de visée de l'ordre de 5 m, la zone de vision intermédiaire correspondant à des distances de visée de l'ordre de 1 à 1,5 m et la zone de vision de près correspondant à des distances de visée de l'ordre de 30 à 40 cm. Physiologiquement, le phénomène d'accommodation est basé sur une déformation contrôlée du cristallin engendrant une modification de courbure des surfaces de la lentille naturelle que forme le cristallin sous l'effet d'une tension ou d'un relâchement de la zonule. Quand un sujet passe de la vision de loin à la vision de près, l'image rétinienne devient floue et le cercle de diffusion maculaire déclenche une contraction réflexe de la portion circulaire du muscle ciliaire. Cette contraction du muscle ciliaire relâche les tensions de la zonule et laisse le cristallin s'arrondir, avec un accroissement du diamètre sagittal et une diminution du diamètre frontal se traduisant par une augmentation de la puissance sphérique du cristallin. A l'inverse, lorsque l'oeil met au point au loin ou à l'infini, le muscle ciliaire est relâché et, la zonule étant sous tension, la lentille s'aplatit.

La fonction accommodative de l'oeil peut se voir affectée à deux principales occasions.

De manière quasi systématique tout d'abord, il est un fait qu'au cours de la vie d'un individu, sa capacité d'accommodation visuelle diminue, si bien que la plupart des personnes ayant franchi la quarantaine ont des besoins de corrections de puissance sphérique différentes en vision de loin et en vision de près. C'est la presbytie. Pour mémoire, il est rappelé que la presbytie n'est pas une amétropie, mais se surajoute à l'éventuelle amétropie préexistante du patient.

Pour palier sa déficience d'accommodation, le presbyte doit recourir à un ou plusieurs équipements visuels qui, outre la correction de ses éventuels amétropie et astigmatisme, réalisent une compensation de cette déficience. Plusieurs solutions s'offrent alors à lui. Il peut porter, soit plusieurs paires de lunettes à lentilles de champ simple foyer parmi lesquelles il choisit celle qui est adaptée à la distance de visée qui l'intéresse à un instant donné, soit une paire de lunettes unique à lentilles de champ multifocales de préférence progressives, soit encore une paire de lentilles de contact, c'est-à-dire des lentilles d'ouverture, multifocales.

On sait qu'une lentille multifocale possède une pluralité de foyers ou puissances sphériques qui diffèrent en fonction de la zone considérée de la lentille. Ainsi, dans les verres de lunettes multifocaux, lentilles de champ, la puissance varie en fonction de la direction du regard, avec vision de loin dans le haut du verre et vision de près en bas. Dans les lentilles de contact multifocales, la multiplicité des foyers procure une profondeur de focalisation ou caustique rendant au système visuel global du patient une capacité de pseudo accommodation. Il se forme en effet concomitamment sur la rétine plusieurs images issues des différentes zones de vision de loin, de près et éventuellement intermédiaire, qui possèdent des puissances dioptriques différentes. Le principe d'adaptation à ce type de lentille est fondé sur la discrimination cérébrale de ces images multiples.

Lorsque le choix se porte sur de telles lentilles multifocales, qu'il s'agisse de lentilles de contact ou de verres de lunettes, celles-ci doivent dans tous les cas présenter une addition correspondant au besoin complet du porteur, puisqu'elles devront palier à elles seules son défaut d'accommodation. Il en résulte que, si le défaut d'accommodation est prononcé, l'addition du verre correcteur d'accommodation devra être forte. Or, les travaux d'investigation de la demanderesse l'ont amené à considérer la force de l'addition d'une lentille comme un facteur de son rejet ou tout au moins comme un frein à l'adaptation du porteur. Cela semble particulièrement vérifié pour les lentilles de contact.

Le patient presbyte peut aussi envisager le port de lentilles de contact (donc d'ouverture) simple foyer chargées de corriger son amétropie en vision de loin ou en vision de près, en combinaison, pour le reste du domaine visuel, avec une paire de lunettes possédant des lentilles (de champ) à foyer simple. On obtient dans ce cas un système hybride combinant les deux types de lentilles, de champ et d'ouverture. On observera toutefois que, dans ce cas, les deux types de lentilles sont à foyer simple.

Outre ce trouble physiologique de la presbytie, il arrive que l'oeil souffre de troubles de réfraction ou même de pathologies plus prononcées, conduisant l'ophtalmologiste à recourir à la chirurgie pour implanter une lentille intraoculaire, après, le cas échéant, ablation du cristallin de l'oeil malade (l'oeil ainsi dépourvu de son cristallin naturel est alors dit aphake). En particulier, le remplacement du cristallin par un implant intraoculaire formant lentille artificielle d'ouverture est devenu une opération courante dans le domaine de la chirurgie de la cataracte ou des fortes amétropies (en particulier les fortes myopies). L'inconvénient en est que l'oeil aphake perd tout pouvoir d'accommodation naturel. Il s'agit alors de rendre au patient opéré une capacité d'accommodation ou tout au moins d'y apporter un palliatif (on parle de compensation). A cet effet, plusieurs solutions ont pu être alternativement proposées.

Une première solution consiste à implanter une lentille monofocale rigide (lentille d'ouverture) ne procurant au système visuel du patient aucune capacité d'accommodation et à prévoir conjointement le port d'un équipement visuel externe du type paire de lunettes (lentille de champ). Le chirurgien et le patient doivent alors choisir, au moment du calcul de la puissance de l'implant intraoculaire, entre vision de près ou vision de loin. Pour le reste du domaine de vision, une correction complémentaire est indispensable et il faut donc équiper le patient d'une ou plusieurs paires de lunettes ayant des lentilles à foyer(s) simple ou multiples. Par exemple, si le patient est implanté avec une lentille intraoculaire monofocale calibrée pour la vision de loin, il est incapable de la moindre accommodation en vision intermédiaire ou de près. Pour accéder à cette partie de domaine visuel, le patient doit avoir recours au port d'au moins deux paires de lunettes à lentilles simple foyer, ou d'une paire de lunettes à lentilles multifocales de préférence progressives. Dans ce dernier cas de figure, qui peut être considéré comme le plus pratique pour le porteur, les lentilles multifocales de champ de la paire de lunettes devront présenter une forte addition, puisqu'elles devront palier à elles seules la perte totale de capacité d'accommodation naturelle du patient. Or, les études de la demanderesses l'ont amené à considérer que, combiné au bouleversement occasionné par l'intervention et l'implant artificiel lui-même, le plus souvent chez des patients assez âgés, le port de lentilles progressives de forte puissance risque d'être difficilement toléré par le porteur.

Une deuxième solution consiste à implanter une lentille intraoculaire dite accommodative, capable de se déformer ou de se déplacer sous l'action du muscle ciliaire pour adapter son foyer au besoin visuel du patient implanté et restaurer ainsi la fonction accommodative naturelle exercée par le cristallin. Les résultats obtenus avec ce type d'implants sont variables et ne font en tout état de cause pas l'objet de la présente invention.

Une troisième solution consiste à implanter une lentille intraoculaire multifocale censée palier seule l'incapacité d'accommodation du patient, sans qu'il soit nécessaire de recourir à un équipement visuel externe. La fonction de ces implants multifocaux est identique à celle d'une lentille de contact multifocale, puisqu'il s'agit dans les deux cas de lentilles d'ouverture. La multiplicité des foyers d'une telle lentille implantée procure une profondeur de focalisation ou caustique rendant au système visuel global du patient une capacité de pseudo accommodation. Le but poursuivi par l'implantation de telles lentilles multifocales est évidemment d'affranchir le patient du port d'une ou plusieurs paires de lunettes, ce qui peut paraître en soi séduisant. Mais on constate en pratique un fort taux d'échec de ces implants multifocaux, soit que l'acuité visuelle soit insuffisante notamment en lecture de près, soit que la fonction pseudo accommodative des implants se révèle inopérante. Il faut alors remplacer l'implant ou accepter la perte d'acuité sans contrepartie de compensation accommodative.

L'observation de cet état de l'art de la compensation des troubles de l'accommodation fait ressortir un problème persistant d'intolérance ou du moins de difficulté d'adaptation des sujets corrigés vis-à-vis des systèmes ou équipements correcteurs qui leurs sont proposés.

Surtout, l'utilisation de lentilles d'ouverture multifocales de forte addition, visant à couvrir seules l'ensemble de la plage d'accommodation, révèle des effets secondaires lourds :

- longueur de la période d'adaptation, pour que le cerveau apprenne à sélectionner l'image nette, voire échec de cette adaptation, avec notamment perception d'un scotome central, d'une vision double ou d'un saut d'images ;

- halos ou éblouissements nocturnes, en rapport avec les aberrations sphériques créées par la défocalisation d'une partie des rayons lumineux;
- perte de luminosité et de sensibilité aux contrastes, liée à une partition des rayons lumineux entre les différentes focales de la lentille.

Pourtant, en pratique quotidienne, le port de lentilles d'ouverture (intraoculaires ou de contact) est a priori la solution préférée des patients du fait de son confort et de sa souplesse de mise en oeuvre.

Le recours à des lentilles de champ progressives n'est pas non plus toujours satisfaisant, selon le trouble du porteur et son besoin d'addition. La progression continue de puissance sphérique nécessite de raccorder, par exemple sur la face avant de la lentille, une zone de vision de loin de plus grand rayon de courbure et une zone de vision de près de plus petit rayon et donne de ce fait inévitablement naissance, dans les parties latérales du verre, à des zones de moindre qualité optique. La disposition de ces zones sur le verre définit le champ de vision nette utilisable par le porteur. Il faut alors trouver un compromis entre largeur de champ et distorsion périphérique. Car tout élargissement du champ de vision nette implique une augmentation des distorsions périphériques, ce qui est gênant en vision statique et surtout dynamique ; et il n'est inversement possible de limiter le niveau de distorsion en périphérie qu'au préjudice du champ de vision nette, qui s'amoindrit. On comprend que cette problématique est d'autant plus délicate et préjudiciable à l'adaptation du porteur que l'addition de puissance de la lentille est élevée.

OBJET DE L'INVENTION

Le but de la présente invention est de proposer une solution alternative pour la compensation des anomalies de l'accommodation, qui favorise à la fois le confort quotidien de port et les chances d'adaptation et de tolérance du sujet, en particulier dans le cas de la pose d'un implant intraoculaire en substitution du cristallin naturel.

A cet effet, on propose selon l'invention un système optique de compensation accommodative, comportant, en combinaison, deux lentilles multifocales, l'une d'ouverture, l'autre de champ. Par multifocale on entend que la lentille possède une pluralité de foyers qui diffèrent selon la zone considérée de celle-ci.

Avantageusement, la lentille d'ouverture présentant une addition de puissance définie comme étant la différence des puissances focales maximum et minimum et la lentille de champ présentant une addition de puissance définie comme étant la différence des puissances focales aux points de référence pour la vision de loin et de près, le système présente une addition globale, définie comme étant la somme des additions des deux lentilles, comprise entre 2,5 et 4 dioptries et l'addition de chaque lentille est supérieure ou égale au quart de l'addition globale.

L'invention offre ainsi une solution qui permet et impose une réduction de l'addition de chaque lentille. Le système optique selon l'invention réalise en effet une répartition de l'addition de puissance globale, dont le porteur a besoin, entre la lentille de champ et la lentille d'ouverture. Il en résulte un élargissement des champs perçus et une réduction des distorsions. Il en résulte également une moindre dépendance au diamètre de la pupille, ce qui est particulièrement avantageux pour la correction visuelle des patients âgés dont la pupille a souvent tendance à rétrécir.

DESCRIPTION DETAILLEE D'UN EXEMPLE DE REALISATION

D'autres caractéristiques et avantages de l'invention apparaîtront à la lecture de la description qui suit d'un mode de réalisation particulier, donnée à titre d'exemple non limitatif.

Il sera fait référence aux dessins présentés en annexe, parmi lesquels :

- la figure 1 est une vue en coupe axiale d'un système optique selon un premier exemple de mise en oeuvre de l'invention,
- la figure 2 est une vue en plan, à échelle supérieure, de la lentille de champ progressive de la figure 1, seule ;
- la figure 3 est une vue en plan, à échelle supérieure, de l'implant intraoculaire bifocal de la figure 1, seul ;
- la figure 4 est une vue en coupe axiale, suivant la ligne III-III de la figure 3 ;
- la figure 5 est une vue en coupe axiale d'un système optique selon un deuxième exemple de mise en oeuvre de l'invention ;
- la figure 6 est une vue en plan, à échelle supérieure, de la lentille de champ bifocale de la figure 5, seule ;
- la figure 7 est une vue en plan, à échelle supérieure, de la lentille de contact asphérique de la figure 5, seule ;
- la figure 8 est une vue de détail de la partie VIII de la figure 5.

Dans le cadre de la présente description, on admettra les définitions suivantes.

On définit la fonction optique d'une lentille ophtalmique, qui lui confère son pouvoir de correction ou compensation, par ses propriétés de réfringence sphérique, cylindrique et prismatique. On comprend qu'une telle définition optique revêt une portée plus générale qu'une définition purement surfacique : elle définit dans sa globalité l'effet de réfringence de la lentille sur un rayon lumineux incident, résultant de la somme algébrique des deux réfringences opérées successivement sur les faces avant et arrière de la lentille. Cette définition optique englobe une pluralité, voire une infinité, de combinaisons de paires de surfaces produisant le même effet de réfringence optique global, comme exposé dans le document « Theoritical aspects of concentric varifocal lenses » de W.N. CHARMAN, dans Ophtal. Physiol. Opt. Vol.2 No.1 pages 75-86, 1982, publié par Pergamon Press pour le British College of Ophtalmic Opticians.

Dans le cadre de la présente invention, on s'intéresse essentiellement à la puissance sphérique. La « puissance de réfringence sphérique » d'une lentille pour un rayon incident traversant cette lentille, également appelée puissance totale ou puissance réfringente ou puissance de focalisation moyenne ou puissance sphérique, est la grandeur qui caractérise et quantifie le premier effet de réfringence sphérique (effet « *loupe* ») de la lentille sur le rayon considéré : si elle est positive, la lentille a un effet convergent sur le rayon ; si elle est négative, l'effet sur le rayon est divergent.

Les valeurs numériques de cette grandeur optique utilisées dans les définitions sont obtenues par la méthode de mesure optique dite de frontofocométrie bien connue des opticiens et précisément décrite notamment dans le document « Paraxial Optics » de W.F. Long, dans Visual Optics and Instrumentation, Ed. N. Charman, Macmillan Press, London 1991, pages 418-419.

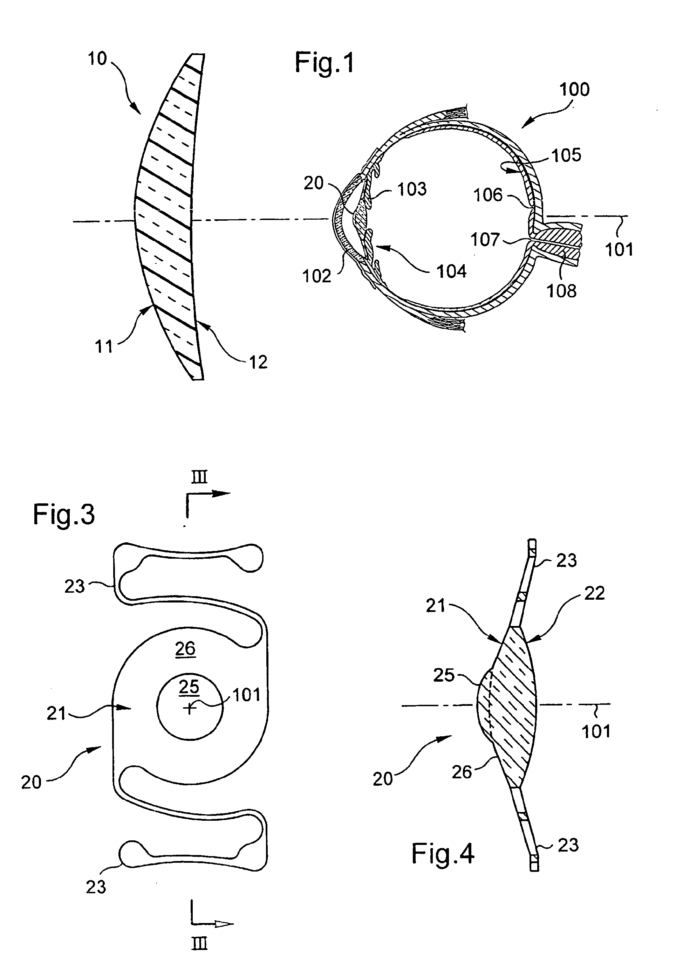

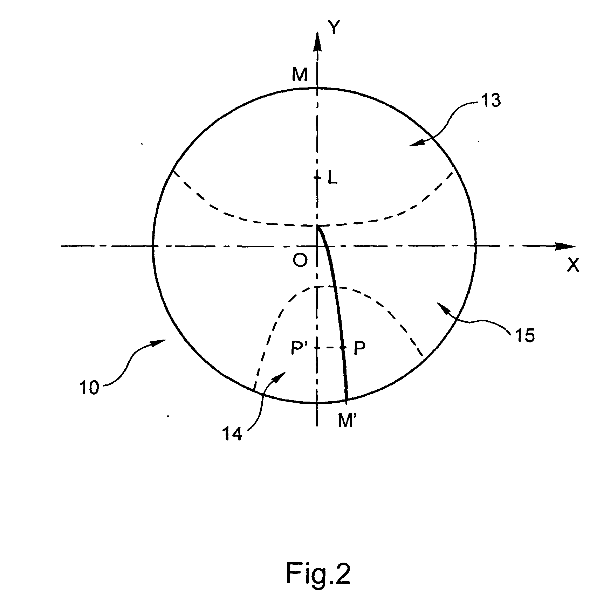

Dans le premier exemple illustré par la figure 1, on propose un équipement visuel pour un patient ayant au moins un oeil aphake 100.

L'oeil 100 possède un axe 101 qui peut être son axe géométrique ou son axe optique. Conventionnellement, on définit l'axe géométrique comme étant la ligne fictive qui traverse l'oeil du pôle antérieur au pôle postérieur et constitue un axe de révolution ou de symétrie de la sphère oculaire de rayon 12 mm. L'axe optique se définit quant à lui comme étant la ligne fictive qui joint les centres de courbure des quatre dioptres principaux de l'oeil constitués par les faces antérieure et postérieure de la cornée et du cristallin (avant ablation de ce dernier). Sur l'axe optique se trouvent les plans principaux, les points nodaux et le centre de rotation de l'oeil. Il coupe par ailleurs la rétine entre la fovéa et la papille.

On reconnaît en outre sur la figure 1, les principaux composants de l'oeil: la cornée 102, l'iris 103, le sac 104 ayant initialement contenu le cristallin, et, au fond de l'oeil, la rétine 105 avec la fovéa 106 située audessus de la papille 107 en regard de laquelle pointe le nerf optique 108.

L'équipement visuel suivant l'invention se compose d'une part d'une paire de lunettes comprenant deux lentilles de champ portées par une monture (non représentée) et d'autre part d'une lentille intraoculaire ou d'une paire de lentilles intraoculaires selon que l'aphakie affecte un seul oeil ou les deux yeux du patient. Lorsque l'aphakie affecte un seul oeil, l'équipement comporte une seule lentille intraoculaire à implanter dans l'oeil aphaque pour former avec la lentille de lunettes associée à l'oeil aphaque un seul système optique selon l'invention, pour la correction de cet oeil aphaque. Lorsque l'aphakie affecte les deux yeux, l'équipement comporte deux lentilles intraoculaires à implanter l'une dans un oeil et l'autre dans l'autre oeil, pour former avec les deux lentilles de lunettes deux systèmes optiques selon l'invention pour la correction de l'un et l'autres des deux yeux aphaques.

Le ou chaque système optique comporte ainsi, en combinaison sur l'axe 101 de l'oeil à corriger 100, deux lentilles 10, 20.

La lentille 10 est montée sur la monture de lunettes et, disposée en regard et à distance de l'oeil 100, est du type lentille de champ tel que défini en introduction.

La lentille de champ 10 est de plus multifocale. Comme évoqué précédemment, on entend par multifocale que la lentille possède une pluralité de foyers qui diffèrent selon la zone considérée de celle-ci. En l'espèce, la lentille 10 est à addition progressive ; mais ce choix n'est pas limitatif. On distingue en effet deux catégories de lentilles multifocales de champ : celles à discontinuité de puissance et celles à addition progressive. La lentille de champ utilisée dans le système selon l'invention peut appartenir à l'une ou l'autre de ces deux catégories.

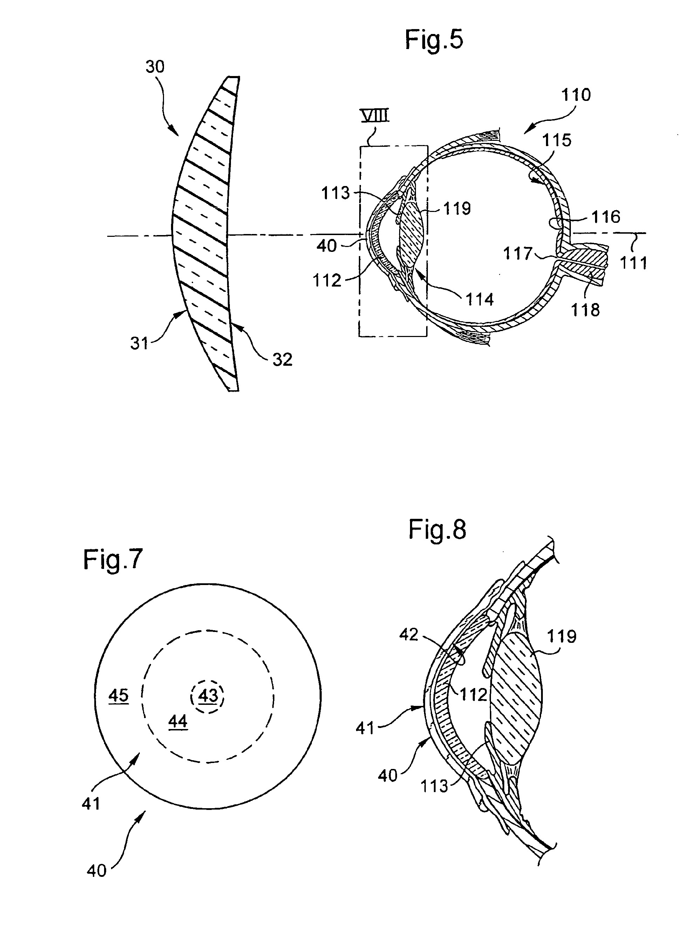

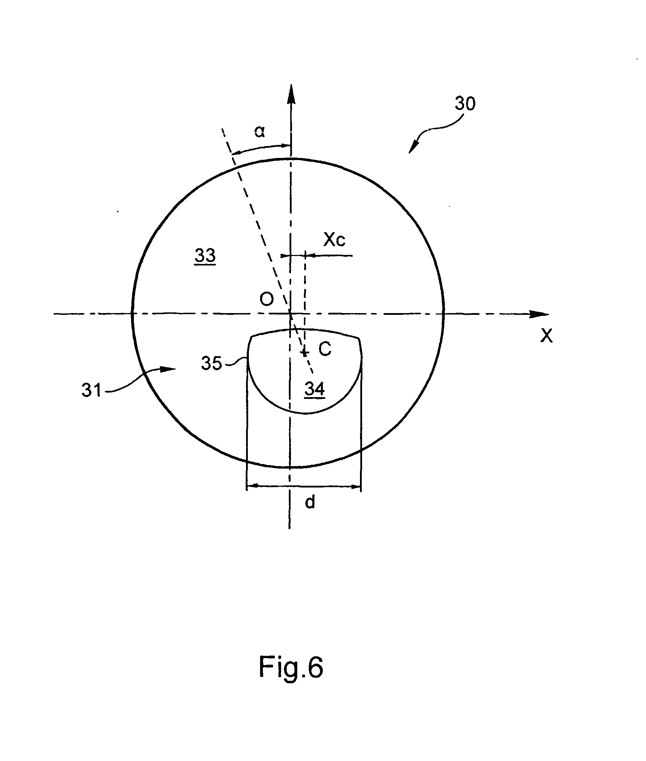

La première catégorie englobe les lentilles possédant un nombre défini de foyers et présentant plusieurs zones discrètes de puissances distinctes uniformes chaque zone, avec une ou plusieurs discontinuités de puissance. Il s'agit typiquement des lentilles bifocales ou trifocales dans lesquelles les zones ou plages de vision de près, intermédiaire et de loin sont séparées par des lignes de discontinuité engendrant un saut d'image. Une lentille de ce type est utilisée dans l'exemple décrit plus loin en référence aux figures 5 et suivantes.

La seconde catégorie de lentilles de champ multifocales est celle des lentilles dites à addition progressive. C'est ce type de lentille qui est utilisé dans l'exemple des figures 1 et 2. De manière générale, une telle lentille possède une infinité de foyers et présente une variation progressive, c'est-à-dire continue et monotone, de puissance. La puissance sphérique augmente de manière continue entre le haut et le bas de la lentille, entre une zone supérieure destinée à la vision de loin et une zone inférieure destinée à la vision de près. La progression peut être portée en face avant (convexe) ou en face arrière (concave), ou encore être répartie sur les deux faces de la lentille. Cette progression est obtenue par une variation continue du rayon de courbure moyen de l'une et/ou l'autre des deux faces de la lentille qui devient de plus en plus petit en face avant et/ou de plus en plus grand en face arrière, vers le bas de la lentille : la surface progressive se cambre en face avant et/ou s'étire en face arrière. La progression de puissance peut aussi être obtenue par tout autre moyen connu.

La progression de puissance de la lentille se fait le long d'une ligne, droite, brisée ou courbée, appelée méridienne principale de progression dont une portion (voire la totalité) est inclinée de quelques degrés, par exemple d'environ 7 à 10 degrés, par rapport à !a verticale. La lentille présente une addition de puissance définie classiquement comme étant la différence des puissances focales (i. e. sphériques) aux points de référence pour la vision de loin et pour la vision de près.

Les lentilles de champ progressives sont désormais bien connues et les détails de leur constitution et fabrication ont fait l'objet de nombreuses publication et commercialisation, notamment de la part de la demanderesse. On pourra, par exemple, utiliser l'une des lentilles commercialisées par la demanderesse sous les marques « Varilux Comfort » et « Varilux Panamic ». On pourra également se référer aux descriptions de lentilles données dans les documents suivants, qui émanent également de la demanderesse :

- brevet français FR-2683642 ou son correspondant américain US-5270745,
- brevet français FR-2683643 ou son correspondant américain US-5272495,
- brevet français FR-2699294 ou son correspondant américain US-5488442,
- brevet français FR-2769998 ou son correspondant américain US-5949519,
- brevet français FR-2769999 ou son correspondant européen EP-0911672,
- brevet français FR-2770000 ou son correspondant européen EP-0911670.

Dans l'exemple illustré par les figures 1 et 2, la lentille 10 consiste, donc, en une telle lentille à addition progressive. Elle possède une face avant 11 convexe qui présente une surface complexe portant la progression de puissance et une face arrière 12 concave, qui est sphérique ou torique et porte la prescription.

En référence plus spécialement à la figure 2, on reconnaît ainsi sur la lentille 10, une zone de vision de loin 13 située dans sa partie supérieure, une zone de vision de près 14 située dans sa partie inférieure et une zone de vision intermédiaire 15 située entre ces deux zones. Rappelons que ces zones et les définitions qui suivent se rapportent aux puissances optiques générées globalement par la lentille, et résultant de la combinaison des géométries de ses deux faces.

Dans la suite de la présente description, on utilise un système de coordonnées orthonormé représenté sur la figure 2. Sur cette figure, on a représenté en plan une lentille finie d'une forme circulaire, avant détourage, qui présente typiquement un diamètre de 60 à 80 mm. Un point de la lentille est utilisé par le laboratoire de prescription pour la référence de prisme ; il s'agit généralement, comme en l'espèce, du centre géométrique de la lentille, noté O sur la figure. Le point de référence prisme est habituellement matérialisé sur la lentille par une marque visible. Le point de référence prisme est utilisé par le laboratoire de prescription pour adapter la lentille au prisme prescrit au porteur par l'ophtalmologiste.

L'axe des abscisses X correspond à l'axe horizontal de la lentille et l'axe des ordonnées Y à l'axe vertical. Dans cette définition, la verticale et l'horizontale correspondent à l'orientation de la lentille lors de son utilisation. Ainsi, dans les lentilles progressives de la demanderesse, l'axe vertical des ordonnées Y est défini par le centre O de la lentille (qui est aussi le point de référence prisme) et par la croix de montage, qui sont en pratique tous deux matérialisés sur la lentille.

On distingue également une méridienne principale de progression MM', représentée en trait gras, qui traverse ces trois zones 13, 15 et 14 suivant une direction globalement verticale, en passant par un point de référence de la vision de loin L et un point de référence de la vision de près P. La méridienne principale de progression MM' se présente ici sous la forme d'une ligne brisée, dont la forme peut varier avantageusement en fonction de l'addition de puissance et de la puissance sphérique nominale au point de référence de la vision de loin L, comme décrit par la demanderesse dans le brevet français FR-2683642 correspondant au brevet américain US-5270745.

En l'espèce, comme dans les lentilles déjà commercialisées par la demanderesse, le point L de référence pour la vision de loin est situé sur l'axe des ordonnées Y à 8 mm au-dessus du centre O de la lentille, tandis que le point P de référence pour la vision de près est décalé du côté nasal de 3 mm par rapport à l'axe des ordonnées Y et se projette sur l'axe des ordonnées en un point P' qui est situé 14 mm en dessous du centre O. Ainsi, les coordonnées du point L sont $X_L = 0$ mm et $Y_L = 8$ mm. Les coordonnées du point P sont $X_P = 3$ mm et $Y_P = 14$ mm.

Pour ce qui est de sa fonction optique, la lentille à addition progressive 10 se définit notamment par deux grandeurs optiques principales : l'addition et la puissance nominale.

L'addition de puissance sphérique $\Delta Pu_{10}$ est égale à la variation de puissance sphérique entre le point de référence L de la zone de vision de loin 13 et le point de référence P de la zone de vision de près 14. Si l'on note $Pu_{10/P}$ la puissance au point P et $Pu_{10/L}$ la puissance au point L, on peut écrire :

$$\Delta Pu_{10} = Pu_{10/P} - Pu_{10/L}$$

La puissance nominale est égale à la puissance $Pu_{10/L}$ au point de référence L de la zone de vision de loin.

Nous verrons que, selon l'invention, l'addition $\Delta Pu_{10}$ doit satisfaire certains critères.

L'oeil à corriger 100 étant aphake, l'autre lentille 20 du système illustré par la figure 1 est une lentille intraoculaire à implanter dans l'oeil aphake 100 en substitution du cristallin naturel, comme illustré par la figure 1. Conformément à la définition donnée en introduction, il s'agit donc d'une lentille d'ouverture.

Dans la forme de mise en oeuvre représentée, la lentille intraoculaire 20 est implantée dans la chambre antérieure de l'oeil 100, c'est-à-dire dans la partie de celui-ci qui s'étend entre sa cornée 102 et son iris 103. Ce mode d'implantation n'est cependant pas limitatif : on pourra implanter la lentille en d'autres emplacements suivant les diverses techniques opératoires connues, notamment dans la chambre postérieure ou le sac capsulaire.

Pour sa fixation dans l'oeil, la lentille 20 formant implant est équipée, à sa périphérie, en positions diamétralement opposées, de deux bras élastiquement déformables 23 en forme générale de « S », propres à son appui sur les corps ciliaires de l'oeil 100, à la racine de l'iris 103.

La lentille 20 est classiquement réalisée en matière synthétique, par exemple en polymère de méthacrylate de méthyle.

La lentille d'ouverture 20 est de plus multifocale. Comme précédemment, on entend par multifocale que la lentille possède une pluralité de foyers qui diffèrent selon la zone considérée de celle-ci. La lentille d'ouverture 20 présente une addition de puissance définie comme étant la différence des puissances focales maximum et minimum.

En l'espèce, dans l'exemple illustré par les figures 1 à 4, la lentille 20 est du type bifocale concentrique à deux zones ; mais ce choix n'est pas limitatif et d'autres types d'implants multifocaux pourront être utilisés, comme nous le verrons plus en détail ultérieurement.

En référence plus spécialement aux figures 3 et 4, cette lentille intraoculaire 20 présente une face antérieure 21 et une face postérieure 22. Dans l'exemple illustré, la face antérieure 21 et la face postérieure 22 sont convexes ; mais d'autres configurations sont envisageables, en particulier celle dans laquelle la face antérieure 21 serait convexe et la face postérieure 22 concave.

Les principes de constitution et la fabrication de telles lentilles intraoculaires multifocales sont aujourd'hui bien connues. Il suffit de rappeler qu'une telle lentille multifocale d'ouverture possède, dans sa partie centrale optiquement utile, plusieurs zones réfractives ou diffractives se distinguant par leurs distances focales. Ces zones de puissances distinctes créent ainsi pour chaque objet plusieurs images différentes superposées sur la rétine, l'une nette les autres floues. Le fonctionnement d'un tel système repose sur les capacités de neutralisation qui permettent au cerveau de sélectionner l'image nette.

Dans l'exemple illustré par les figures 2 et 3, la lentille intraoculaire 20 est bifocale concentrique. Elle possède une zone centrale 25 pour la vision de près de puissance $Pu_{20/P}$ et une zone annulaire périphérique 26 pour la vision de loin de puissance $Pu_{20/L}$. L'addition de la lentille 20, notée $\Delta Pu_{20}$, est la différence entre ces deux puissances :

$$\Delta Pu_{20} = Pu_{20/P} - Pu_{20/L}$$

Selon un aspect essentiel de l'invention, les additions $\Delta Pu_{10}$ et $\Delta Pu_{20}$ des deux lentilles 10 et 20 sont dépendantes l'une de l'autres et doivent satisfaire certains critères.

Considérons que le système optique formé par la combinaison des deux lentilles 10 et 20 présente une addition globale, définie comme étant la somme des additions des deux lentilles. Cette addition globale correspond, à 10% près, à l'addition prescrite au porteur. Elle est, selon l'invention, d'une part limitée et d'autre part répartie sur les deux lentilles 10 et 20.

Plus précisément, cette addition globale est comprise entre 2,5 et 4 dioptries, d'où l'expression :

$$2,5 \text{ dpt} \leq \Delta Pu_{10} + \Delta Pu_{20} \leq 4 \text{ dpt}$$

S'agissant en l'espèce d'un oeil aphake, elle pourra avantageusement être choisie proche de 4 dioptries.

Conjointement, l'addition de chacune des deux lentilles 10 et 20 est supérieure ou égale au quart de l'addition globale, ce qui garantit l'équilibre et l'efficacité de la répartition :

$$\Delta Pu_{10} \geq (\Delta Pu_{10} + \Delta Pu_{20}) / 4$$

et

$$\Delta Pu_{20} \geq (\Delta Pu_{10} + \Delta Pu_{20}) / 4$$

De préférence, la répartition d'addition entre les deux lentilles pourra être paritaire. C'est ainsi que, dans l'exemple proposé prévoyant une addition globale de 4 dioptries, on pourra donner à la lentille de lunettes 10 comme à la lentille intraoculaire 20 une même addition d'environ 2 dioptries.

Les figures 5 à 8 illustrent un deuxième exemple de mise en oeuvre de l'invention. Il s'agit d'un équipement visuel pour un patient presbyte. Mais le même équipement pourrait également équiper un patient ayant perdu tout pouvoir d'accommodation naturelle, tel qu'un aphake.

L'équipement se compose d'une part d'une paire de lunettes avec une lentille disposée en regard de chaque oeil et d'autre part d'une paire de lentilles de contact cornéennes apposées chacune sur un oeil. On forme ainsi deux systèmes optiques selon l'invention constitués chacun de la lentille de lunettes (lentille de champ) et de la lentille de contact (lentille d'ouverture) placées en regard d'un même oeil.

Sur la figure 5, on reconnaît un oeil 110 avec ses principaux composants : cornée 112, l'iris 113, sac 114 contenant le cristallin 119, et, au fond de l'oeil, la rétine 115 avec la fovéa 116 située au-dessus de la papille 117 en regard de laquelle pointe le nerf optique 118.

Chaque système optique comporte, en combinaison sur l'axe 101 de l'oeil à corriger 100, deux lentilles 30, 40.

La lentille 30 est montée sur la monture de lunettes (non représentée) et, disposée en regard et à distance de l'oeil 100, est du type lentille de champ tel que défini en introduction.

La lentille de champ 30 est de plus multifocale, conformément à la définition précédemment donnée. Plus précisément, la lentille 10 appartient ici à la première catégorie des lentilles de champ multifocales définie plus haut. Il s'agit donc d'une lentille à discontinuité de puissance. Mais ce choix n'est pas limitatif et on pourra utiliser une lentille à addition progressive en combinaison avec une lentille de contact.

Les lentilles de champ à discontinuité de puissance sont bien connues et il n'est pas nécessaire d'en expliciter ici les détails de constitution et de fabrication qui ont fait l'objet de nombreuses publications et commercialisations. Il suffit de rappeler qu'il existe deux principaux types de lentilles multifocales à discontinuité de puissance qui conviennent indifféremment à la mise en oeuvre de la présente invention : les lentilles taillées et les verres fusionnés. La multiplicité de puissance pour un même verre peut en effet s'obtenir, soit par un changement de courbure d'une des deux faces (multifocal discontinu taillé ou moulé), soit par l'inclusion d'un matériau d'indice de réfraction plus élevé (multifocal fusionné). On pourra, par exemple, utiliser l'une des lentilles commercialisées par la demanderesse sous les marques « CT 28 ORMEX », « CT 28 ORMA », « TRIFOCAL 22x36 », « TELEMIL », « TELEX », « TELARC ».

Dans l'exemple illustré par les figures 5 et 6, la lentille 30 consiste, donc, en une telle lentille à discontinuité de puissance. Elle possède une face avant 31 convexe qui porte la discontinuité de puissance et une face arrière 32 concave qui est sphérique ou torique et porte la prescription.

Sur la figure 6, on a représenté en plan une lentille finie dont le contour, avant détourage, est de forme circulaire et présente typiquement un diamètre de 60 à 80 mm. L'axe des abscisses X correspond à l'axe horizontal de la lentille et l'axe des ordonnées Y à l'axe vertical. Dans cette définition, la verticale et l'horizontale correspondent à l'orientation de la lentille lors de son utilisation.

La face avant 31 de la lentille 30 comporte deux zones sphériques de centres et de courbures distincts. On distingue ainsi une zone principale 33 située dans la partie supérieure de la lentille et servant à la vision de loin. Le centre optique de cette zone 33 est ici confondu avec le centre géométrique O de la lentille, mais peut aussi être décalé d'environ 1 mm du côté nasal sur l'axe des abscisses X.

A l'intérieure de la zone de vision de loin 33, dans la partie inférieure de la lentille, se détache, délimitée par une ligne de discontinuité 35, une zone 34 servant à la vision de près. Cette zone, de plus forte courbure que la zone 32, confère localement à la lentille une plus forte puissance sphérique. Le centre optique de cette zone 34, noté C, est décalé du côté nasal de quelques millimètres. En l'espèce, la droite OC forme un angle_= 10 degrés avec l'axe des ordonnées Y et l'abscisse du centre C vaut $Xc = 3$ mm. La ligne 35 délimitant la zone 34 présente la forme d'une portion de cercle de diamètre 25 mm et de centre C, tronquée dans sa partie supérieure par une portion globalement horizontale, qui est ici légèrement courbée comme illustré par la figure 6 et dont le sommet est situé quelques millimètres (en l'espèce, 4,5 mm) en dessous de l'axe X.

Pour ce qui est de sa fonction optique, la lentille bifocale 30 se définit notamment par deux grandeurs optiques principales : l'addition et la puissance nominale.

L'addition de puissance sphérique $\Delta Pu_{30}$ est égale à la différence entre les puissances sphériques des zones de vision de loin 33 et de vision de près 34. Si l'on note $Pu_{30/L}$ la puissance dans la zone de vision de loin et $Pu_{30/P}$ la puissance dans la zone de vision de près, on peut écrire :

$$\Delta Pu_{30} = Pu_{30/P} - Pu_{30/L}$$

La puissance nominale est égale à la puissance de la zone de vision de loin $Pu_{30/L}$.

Nous verrons que, selon l'invention, l'addition $\Delta Pu_{30}$ doit satisfaire certains critères.

L'autre lentille 40 du système illustré par la figure 5 est une lentille de contact cornéenne à apposer contre la cornée 112 de l'oeil 110, comme illustré. Conformément à la définition donnée en introduction, il s'agit donc d'une lentille d'ouverture.

La lentille 40 est une lentille souple classiquement réalisée en matière synthétique hydrophile, par exemple : hydroxyethyl methacrylate, acrylmonomer, vinylpyrrolidone (N-vinyl 2-pyrrolidone), epoxy.

La lentille de contact 40 est de plus multifocale. Comme évoqué précédemment, on entend par multifocale que la lentille possède une pluralité de foyers qui diffèrent selon la zone considérée de celle-ci. Elle présente une addition de puissance définie comme étant la différence de ses puissances focales maximum et minimum.

Les principes de constitution et de fabrication de telles lentilles de contact multifocales sont aujourd'hui bien connus. Il suffit de rappeler qu'une telle lentille multifocale d'ouverture possède, dans sa partie optiquement utile, plusieurs zones réfractives ou diffractives se distinguant par leurs distances focales. Ces zones de puissances distinctes créent ainsi pour chaque objet plusieurs images différentes superposées sur la rétine, l'une nette les autres floues. Le fonctionnement d'un tel système repose sur les capacités de neutralisation qui permettent au cerveau de sélectionner l'image nette.

En l'espèce, la lentille 40 est du type asphérique progressive ; mais ce choix n'est pas limitatif, comme nous le verrons plus en détail ultérieurement.

La lentille de contact 40 possède une face antérieure 41 et une face postérieure 42. La face antérieure 41 présente une géométrie sphéro-asphérique, avec une zone centrale sphérique 43 pour la vision de près entourée par une zone annulaire à profil asphérique 44 qui génère la progression de puissance, de la périphérie vers le centre, pour les visions intermédiaires et de loin. Cette progression de puissance est continue et monotone. Une zone périphérique 45 prolonge la lentille pour son assise et son maintien sur l'oeil, sans exercer de fonction optique. La zone centrale 43 présente un diamètre de 2 à 4 mm et la zone annulaire 43 présente un diamètre de 6 à 14 mm. Le raccordement entre les zones est continu au second ordre au moins. Il est entendu que les cercles pointillés délimitant ces trois zones ont été portés sur la figure 7 à des fins didactiques et n'ont aucune consistance physique visible. La face postérieure 42 est sphérique ou torique et est adaptée classiquement à la prescription du porteur.

Soit $Pu_{40/P}$ la puissance de la lentille dans la zone centrale pour la vision de près 43 et $Pu_{20/L}$ la puissance pour la vision de loin, qui correspond à la puissance minimum de la lentille dans la zone annulaire asphérique pour la vision de loin 44 et qui est atteinte au bord extérieur de cette zone.

L'addition de la lentille 40, notée $\Delta Pu_{20}$, est la différence entre ces deux puissances :

$$\Delta Pu_{40} = Pu_{40/P} - Pu_{40/L}$$

Selon un aspect essentiel de l'invention, les additions $\Delta Pu_{30}$ et $\Delta Pu_{40}$ des deux lentilles 30 et 40 sont dépendantes l'une de l'autres et doivent satisfaire certains critères.

Considérons que le système optique formé par la combinaison des deux lentilles 30 et 40 présente une addition globale, définie comme étant la somme des additions des deux lentilles. Cette addition globale correspond, à 10% près, à l'addition prescrite au porteur. Elle est, selon l'invention, d'une part limitée et d'autre part répartie sur les deux lentilles 30 et 40.

Plus précisément, cette addition globale est comprise entre 2,5 et 4 dioptries, d'où l'expression :

$$2,5 \text{ dpt} \leq \Delta Pu_{30} + \Delta Pu_{40} \leq 4 \text{ dpt}$$

S'agissant en l'espèce d'un oeil non aphake souffrant d'un presbytie prononcée, elle pourra avantageusement être choisie proche de 3 dioptries.

Conjointement, l'addition de chacune des deux lentilles 30 et 40 est supérieure ou égale au quart de l'addition globale, ce qui garantit l'équilibre et l'efficacité de la répartition :

$$\Delta Pu_{30} \geq (\Delta Pu_{30} + \Delta Pu_{40}) / 4$$

et

$$\Delta Pu_{40} \geq (\Delta Pu_{30} + \Delta Pu_{40}) / 4$$

De préférence, la répartition d'addition entre les deux lentilles pourra être paritaire. C'est ainsi que, dans l'exemple proposé prévoyant une addition globale de 3 dioptries, on pourra donner à la lentille de lunettes 30 comme à la lentille intraoculaire 40 une même addition d'environ 1,5 dioptries.

L'invention n'est pas limitée aux modes de réalisation qui viennent d'être décrits. En particulier, la lentille de champ multifocale 30 peut affecter toute forme connue. Les exemples des figures 1 et 2 pour la lentille progressive et des figures 5 et 6 pour la lentille bifocale discontinue ne sont pas limitatifs.

De même, la lentille d'ouverture multifocale peut, quelque soit l'origine du trouble à corriger, affecter toute forme connue. Les exemples des figures 3 et 4 pour la lentille intraoculaire et des figures 7 et 8 pour la lentille de contact ne sont pas limitatifs. Il est possible d'utiliser, pour la mise en oeuvre de l'invention, tout type de lentille d'ouverture multifocale décrit dans l'état de la technique. Dans cette perspective, il est entendu que l'enseignement fourni pour une lentille de contact pourra être transposé à un usage comme lentille intraoculaire par simple adjonction à la lentille de moyens de sa fixation dans l'oeil.

On distingue ainsi trois catégories de lentilles multifocales d'ouverture : concentriques, asphériques progressives ou diffractives. La lentille d'ouverture utilisée dans le système selon l'invention peut appartenir à l'une quelconque de ces catégories.

La première catégorie englobe les lentilles possédant un nombre défini de foyers et présentant plusieurs zones discrètes de puissances distinctes avec une ou plusieurs discontinuités de puissance. Il s'agit typiquement des lentilles bifocales ou trifocales dans lesquelles les zones de vision de près, intermédiaire (éventuellement) et de loin sont séparées par des lignes de discontinuité engendrant un saut d'image.

C'est ainsi, par exemple, que l'on pourra utiliser une lentille d'ouverture, de contact ou intraoculaire, bifocale concentrique à deux zone telle que celle décrite dans le brevet américain US 3420006. Il s'agit d'une lentille d'ouverture bifocale de révolution (en l'espèce une lentille de contact, mais l'enseignement est transposable à une lentille intraoculaire) possédant une zone centrale ayant une puissance pour la vision de loin, entourée d'une zone annulaire ayant une puissance pour la vision de loin. Les brevets américains US 3270007, US 3726587 et US 4636049 décrivent la configuration inverse correspondant à l'exemple des figures 2 et 3 (pour une lentille de contact), avec une zone centrale pour la vision de près et une zone annulaire périphérique pour la vision de loin.

On pourra aussi utiliser une lentille intraoculaire ou de contact multifocale concentrique multizones. Ces lentilles de révolution possèdent une succession de plusieurs zones annulaires concentriques en plus de la partie centrale, alternant puissance pour la vision de près et puissance pour la vision de loin, comme décrit dans la demande de brevet internationale WO 89/02251 ou dans le brevet américain US 6527389. Les parties de transition entre les anneaux peuvent être adoucies pour présenter une certaine puissance réfractive servant à la vision intermédiaire. Eventuellement, une troisième puissance pour la vision intermédiaire peut être ajoutée à l'alternance.

La seconde catégorie de lentilles d'ouverture multifocales utilisables est celle des lentilles intraoculaires ou de contact asphériques progressives. Il s'agit de lentilles de révolution dans lesquelles la puissance varie progressivement suivant une loi de variation monotone depuis le centre vers la périphérie ou inversement. La courbure asphérique peut être sur la face antérieure ou sur la face postérieure. Bien que la configuration inverse soit envisageable, on préférera porter l'asphéricité sur la face antérieure, de sorte que la zone de vision de près se trouve au centre de la lentille. Différentes géométries sont possibles, en particulier : géométrie sphéro-asphérique, avec un bouton central sphérique se prolongeant en périphérie par un profil asphérique qui génère la progression (ou dégression) de puissance (comme dans l'exemple des figures 7 et 8) ou encore géométrie concentrique asphérique qui fait alterner des zones de vision de loin, de près et intermédiaire, suivant un continuum de surfaces asphériques concentriques. Des exemples de telles lentilles d'ouverture progressives sont donnés dans le brevet anglais GB-2288033 et les brevets américains US-6322213, US-5214453, US-4861152, US-4580882, US-4199231, US-5125729.

Enfin, il existe aussi des lentilles d'ouverture multifocales diffractives. Ces lentilles possèdent sur l'une de leurs faces un réseau diffractif. Il s'agit typiquement d'un réseau d'échelettes concentriques. Dans le cas de lentilles de contact cornéennes, le réseau diffractif est réalisé en face postérieure pour être stabilisé par son immersion dans les larmes et sa profondeur est très faible (de l'ordre de 0,003 mm) pour éviter un effet délétère sur l'épithélium cornéen. Un exemple d'une telle lentille est donné dans le brevet américain US-4162122.

**Revendications**

1. Système optique de compensation accommodative, comportant, en combinaison, deux lentilles multifocales, l'une d'ouverture, l'autre de champ.

2. Système optique selon la revendication 1, dans lequel, la lentille d'ouverture présentant une addition de puissance définie comme étant la différence des puissances focales maximum et minimum et la lentille de champ présentant une addition de puissance définie comme étant la différence des puissances focales aux points de référence pour !a vision de loin et de près, le système présente une addition globale, définie comme étant la somme des additions des deux lentilles, comprise entre 2,5 et 4 dioptries et l'addition de chaque lentille est supérieure ou égale au quart de l'addition globale.

3. Système optique selon la revendication précédente, dans lequel l'addition de chaque lentille est supérieure ou égale 40% de l'addition globale.

4. Système selon l'une des revendications précédentes, dans lequel la lentille de champ appartient à l'une des catégories suivantes : à addition progressive, à discontinuité de puissance.

5. Système selon l'une des revendications précédentes, dans lequel la lentille d'ouverture appartient à l'une des catégories suivantes : concentrique, asphérique progressive, diffractive.

6. Système optique selon l'une des revendications 1 à 3, dans lequel les deux lentilles sont du type progressives.

7. Système optique selon l'une des revendications précédentes, dans lequel, l'oeil à corriger étant aphaque, ladite lentille d'ouverture est une lentille intraoculaire à implanter dans l'oeil en substitution du cristallin naturel.

8. Système optique selon l'une des revendications 1 à 6, dans lequel la lentille d'ouverture est une lentille de contact cornéenne.

9. Equipement visuel pour un patient ayant au moins un oeil aphake, comportant un système optique selon la revendication 7 et se composant d'une part d'une paire de lunettes possédant une lentille disposée en regard de l'oeil concerné et constituant ladite lentille de champ du système et d'autre part de ladite lentille intraoculaire.

10. Equipement visuel pour un patient presbyte, comportant, associé à chaque oeil, un système optique selon la revendication 8 et se composant d'une part d'une paire de lunettes avec une lentille disposée en regard de chaque oeil et constituant ladite lentille de champ du système associé à cet oeil et d'autre part d'une paire de lentilles de contact apposées chacune sur un oeil et constituant lesdites lentilles d'ouverture du système associé à cet oeil.

## Claims

1. An optical system presenting accommodation compensation, the system comprising in combination two multifocal lenses, one of them being an aperture lens and the other a field lens.

2. An optical system according to claim 1, in which the aperture lens presents power addition defined as being the difference between the maximum and minimum focal powers and the field lens presents power addition defined as being the difference between the focal powers at the reference point for far vision and or near vision, the system presenting global addition defined as being the sum of the additions of the two lenses, lying in the range 2.5 diopters to 4 diopters, and the addition of each lens is greater than or equal to one-fourth of the overall addition.

3. An optical system according to the preceding claim, in which the addition of each lens is greater than or equal to 40% of the overall addition.

4. A system according to any preceding claim, in which the field lens belongs to one of the following categories: a lens having progressive addition, a lens having a power discontinuity.

5. A system according to any preceding claim, in which the aperture lens belongs to one of the following categories: a concentric lens, a progressive aspherical lens, a diffractive lens.

6. An optical system according to any one of claims 1 to 3, in which both lenses are of the progressive type.

7. An optical system according to any preceding claim, in which the eye to be corrected is aphakic, and said aperture lens is an intraocular lens for implanting the eye as a replacement for the natural lens.

8. An optical system according to any one of claims 1 to 6, in which the aperture lens is a corneal contact lens.

9. Visual equipment for a patient having at least one aphakic eye, the equipment comprising an optical system according to claim 7 and made up firstly of a pair of spectacles possessing a lens placed in register with the eye in question and constituting said field lens of the system, and secondly of said intraocular lens.

10. Visual equipment for a patient presenting presbyopia, the equipment comprising in association with each eye, an optical system according to claim 8 and made up firstly of a pair of spectacles with a lens placed in register with each eye and constituting said field lens of the system associated with the eye, and secondly of a pair of contact lenses each placed on a respective one of the eyes and constituting said aperture lenses of the system associated with the eye.

## Patentansprüche

1. Optisches akkommodatives Kompensationssystem, das kombiniert zwei Multifokallinsen, nämlich eine Öffnungslinse und eine Feldlinse aufweist.

2. Optisches System nach Anspruch 1, bei dem die Öffnungslinse eine Stärkenhinzufügung aufweist, die als Unterschied der maximalen und minimalen Brennstärke definiert ist, und die Feldlinse eine Stärkenhinzufügung aufweist, die als der Unterschied der Brennstärken an den Referenzpunkten für das Weitsehvermögen und Nahsehvermögen definiert ist, wobei das System eine globale Hinzufügung aufweist, die als die Summe der Hinzufügungen der zwei Linsen definiert ist, die zwischen 2,5 und 4 Dioptrien liegt, und die Hinzufügung jeder Linse größer oder gleich dem Viertel der Gesamthinzufügung ist.

3. Optisches System nach dem vorhergehenden Anspruch, bei dem das Hinzufügen jeder Linse größer oder gleich 40 % der Gesamthinzufügung ist.

4. Optisches System nach einem der vorhergehenden Ansprüche, bei dem die Feldlinse zu einer der folgenden Kategorien gehört: mit allmählichem Hinzufügen, mit Stärkendiskontinuität.

5. Optisches System nach einem der vorhergehenden Ansprüche, bei dem die Öffnungslinse zu einer der folgenden Kategorien gehört: konzentrisch, asphärisch progressiv, diffraktiv.

6. Optisches System nach einem der Ansprüche 1 bis 3, bei dem die zwei Linsen progressive Linsen sind.

7. Optisches System nach einem der vorhergehenden Ansprüche, bei dem, da das zu korrigierende Auge aphak ist, die Öffnungslinse eine intraokulare Linse ist, die in das Auge als Ersatz für die natürliche Augenlinse zu implantieren ist.

8. Optisches System nach einem der Ansprüche 1 bis 6, bei dem die Öffnungslinse eine Hornhautkontaktlinse ist.

9. Sehausstattung für einen Patienten, der mindestens ein aphakes Auge hat, die ein optisches System nach Anspruch 7 aufweist und einerseits aus einer Brille besteht, die eine Linse hat, die gegenüber dem betreffenden Auge angeordnet ist und die Feldlinse des Systems bildet, und andererseits aus der intraokularen Linse.

10. Sehausstattung für einen weitsichtigen Patienten, die jedem Auge zugewiesen ein optisches System nach Anspruch 8 aufweist und einerseits aus einer Brille mit einer Linse besteht, die gegenüber jedem Auge angeordnet ist und die Feldlinse des diesem Auge zugewiesenen Systems ist, und andererseits aus einem Paar Kontaktlinsen, die jeweils auf ein Auge angelegt werden und die Öffnungslinsen des zu diesem Auge gehörenden Systems bilden.

# Fig.1

# Fig.3

# Fig.4

Fig.2

## Fig.5

## Fig.7

## Fig.8

Fig.6